(19) Europäisches Patentamt
European Patent Office
Office européen des brevets

(11) Veröffentlichungsnummer: **0 032 673**
**B1**

(12) # EUROPÄISCHE PATENTSCHRIFT

(45) Veröffentlichungstag der Patentschrift: **21.12.83**

(51) Int. Cl.³: **C 07 D 265/30,** C 07 D 295/02, C 07 D 211/14, A 01 N 43/36, A 01 N 43/40, A 01 N 43/84

(21) Anmeldenummer: **81100066.0**

(22) Anmeldetag: **08.01.81**

(54) **N-(3(4'-tert.Butyl-cyclohex-1'-en-1'-yl)-2-methyl-propyl-1)- substituiertes 2,6-Dimethylmorpholin, Piperidin und Pyrrolidin, ihre Herstellung und Verwendung als Fungizide.**

(30) Priorität: **17.01.80 DE 3001581**

(43) Veröffentlichungstag der Anmeldung: **29.07.81 Patentblatt 81/30**

(45) Bekanntmachung des Hinweises auf die Patenterteilung: **21.12.83 Patentblatt 83/51**

(84) Benannte Vertragsstaaten: **AT BE CH DE FR GB IT LI NL SE**

(56) Entgegenhaltungen:
**EP - A - 0 005 541**
**EP - A - 0 019 764**
**DE - A - 2 752 135**
**US - A - 3 046 280**
**US - A - 4 131 744**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Himmele, Walter, Dr., Eichenweg 14, D-6909 Walldorf (DE)**
Erfinder: **Pommer, Ernst-Heinrich, Dr., Berliner Platz 7, D-6703 Limburgerhof (DE)**

**EP 0 032 673 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# N-[3(4'-tert.Butyl-cyclohex-1'-en-1'-yl)-2-methyl-propyl-1]-substituiertes 2,6-Dimethylmorpholin, Piperidin und Pyrrolidin, ihre Herstellung und Verwendung als Fungizide

Die vorliegende Erfindung betrifft neue wertvolle Cycloalkylamine mit einem 3(4'-tert.-Butyl-cyclohex-1'-en-1'-yl)-2-methyl-1-propyl-)-Substituenten am Stickstoff, sowie deren Salze. Diese Stoffe besitzen einer hervorragende fungicide Wirkung und eignen sich zur Bekämpfung von phythopathogenen Pilzen.

Es ist bekannt, daß man 4 3-(p.-tert.Butyl-phenyl)-2-methyl-propyl-cis-2,6-Dimethyl-morpholin, -piperidin oder -pyrrolidin (DE-OS 2 656 747, DE-OS 2 752 096 und DE-OS 2 752 135) für die Bekämpfung von Mehltau und Rostpilzen im Getreide speziell im Weizen verwenden kann. Anstelle des 2,6-Dimethylmorpholin-Restes lassen sich auch Reste anderer cyclischer Amine wie Piperidin oder 3-Methyl-piperidin, verwenden. Die Dauer der Wirksamkeit dieser Verbindungsklasse innerhalb der ein voller Schutz gegen die Schadpilze erzielt wird, liegt bei drei bis vier Wochen. Eine Wiederholung der Anwendung des Pilzschutzmittels ist oft erforderlich.

Es wurde nun gefunden, daß N 3(4'-tert.Butylcyclohex-1'-en-1'-yl)-2-methyl-propyl-cycloalkylamine der Formel

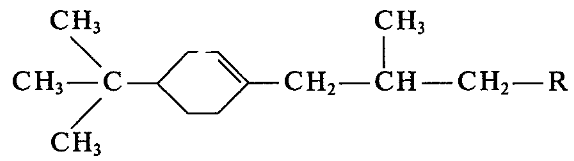

in der R den Rest des 2,6-Dimethylmorpholins, Piperidins oder Pyrrolidins bedeutet, und ihre Salze eine sehr gute fungizide Wirkung haben.

Salze sind beispielsweise die Säureadditionssalze mit anorganischen oder organischen Säuren, z. B. Chloride, Fluoride, Bromide, Jodide, Sulfate, Nitrate, Phosphate, Acetate, Propionate oder mit Säuren von Tensiden, z. B. Dodecylbenzolsulfonsäure.

Die erfindungsgemäßen 2,6-Dimethylmorpholinderivate können als cis- und trans-Isomere isoliert werden.

Die Synthese der neuen Verbindungen erfolgt beispielsweise durch partielle Hydrierung der entsprechenden aromatischen Verbindungen, d. h. der Verbindungen, die anstelle des Cyclohexenrestes den Phenylrest enthalten, mit Wasserstoff in Gegenwart eines Katalysators. Eine partielle Hydrierung gelingt beispielsweise mit oxydischen Rutheniumkatalysatoren. Um eine gute Ausbeute an der Cyclohex-1'-en-1'-yl-Verbindung zu erzielen, kann die Hydrierung nur mit einer begrenzten Wasserstoffmenge vorgenommen werden. Es empfiehlt sich hierbei, zuerst die für eine Überführung der aromatischen Verbindung in das Cyclohexanderivat (Perhydrierung) erforderliche Wasserstoffmenge für den Autoklaven, in dem diese Umsetzung vorgenommen werden soll, zu ermitteln. Für die Herstellung der Cyclohex-1'-en-1'-yl-Verbindung wird zweckmäßig nur etwa 1/4 bis etwa 1/2 der für eine vollständige Hydrierung benötigten Wasserstoffmenge verwendet. Die Hydrierung wird dabei durch Unterbrechung der Wasserstoffzufuhr gestoppt.

Als Lösungsmittel für die Hydrierung können Dioxan, Äther oder cyclische Äther wie Tetrahydrofuran verwendet werden.

Der oxydische Ruthenium-Katalysator wird zweckmäßig durch Fällung des Oxvdhydrats von Ruthenium mit Natronlauge aus einer wäßrigen Lösung von Rutheniumtrichlorid-trihydrat gewonnen. Vom Katalysator werden in der getrockneten Form etwa 0,001 bis 0,5 Gewichtsprozent (bezogen auf das gesamte Reaktionsgemisch) verwendet. Der Katalysator kann nach der Hydrierung abgetrennt und für die nächste Hydrierung wiederverwendet werden.

Die Abtrennung des neuen Cyclohexenderivates muß aus einem Reaktionsgemisch erfolgen, das außer dem gesuchten Produkt noch das 1,4-disubstituierte Cyclohexanderivat neben dem Ausgangsmaterial, nämlich der aromatischen Verbindung enthält. Die Abtrennung dieser beiden Begleitprodukte kann durch fraktionierte Destillation über eine sehr gut wirksame Destillationskolonne erfolgen. Nach der destillativen Abtrennung des Lösungsmittels aus dem Reaktionsgemisch kann zunächst bei der Fraktionierung das cis-1,4-disubstituierte Cyclohexanderivat erhalten werden. Als nächstes Produkt der Fraktionierung erhält man das trans-1,4-disubstituierte Cyclohexanderivat. Als Destillationskolonne wird beispielsweise eine Kolonne mit Edelstahldrahtnetzwendeln als Kolonnenfüllung verwendet. Als nächste höhersiedende Komponente wird das Ausgangsprodukt -- die aromatische Verbindung — erhalten. Als höchstsiedende Komponente wird unter diesen Bedingungen das Cyclohex-1'-en-1-yl-derivat gewonnen. Diese Reihenfolge gilt sowohl bei einem Druck von 2 Torr als auch für einen Druck von 25 Torr jeweils gemessen am Kopf der Destillationskolonne.

## Beispiel 1

### Herstellung von N-[3-(4'-tert.Butyl-1'-cyclohexen-1'-yl)-2-methyl-1-propyl]-cis-2,6-dimethyl-morpholin (1)

In einen 250-ml-Schüttelautoklaven werden 70 g N-[3-p.-tert.-Butylphenyl)-2-methyl-propyl]-cis-2,6-dimethyl-morpholin eingefüllt. Um die Viskosität bei der Hydrierung abzusenken, werden 70 g Dioxan zugegeben. Als Katalysator werden 0,3 g Rutheniumoxydhydrat zugesetzt. Bei der vollständigen Hydrierung dieses Ansatzes im Schüttelautoklaven wird unter sonst gleichen Reaktionsbedingungen im ersten Versuch (1) 205 bar Wasserstoff aufgenommen und bei einem zweiten Versuch (2) 199 bar Wasserstoff. Die gaschromatographische Analyse des Reaktionsgemisches zeigt das Vorhandensein der cis- und der trans-isomeren Cyclohexanverbindung. Das Ausgangsprodukt (II) ist also vollständig hydriert worden.

Zur Herstellung des Cyclohexenderivates wird die Hydrierung unter sonst gleichen Reaktionsbedingungen nach einer Wasserstoffaufnahme von 53 bar abgebrochen (Versuch A). Die Hydrierung erfolgte in allen drei Versuchen unter schrittweisem Anheben der Temperatur und des Druckes. Es wurden die folgenden Daten gemessen:

| Temperatur in °C | Druck in bar Wasserstoff | Vergleichs-Versuch 1 | Vergleichs-Versuch 2 | Versuch A |
|---|---|---|---|---|
| | | | Wasserstoffaufnahme in bar | |
| 80 | 80 | 0 | 0 | 0 |
| 100 | 100 | 7 | 5 | 0 |
| 120 | 120 | 0 | 73 | 53 |
| 140 | 140 | 198 | 15 | – |
| 160 | 160 | 0 | 0 | – |
| 180 | 180 | 0 | 26 | – |
| 200 | 200 | 0 | 80 | – |

Die Analyse des Reaktionsgemisches aus Versuch A zeigte, daß das Reaktionsgemisch 10% (Gew.-%) cis-1,4-Cyclohexanderivat, 6% trans-isomeres Cyclohexanprodukt und 24% der neuen Cyclohexenverbindung neben 60% der Ausgangsverbindung enthält.

## Beispiel 2

Wie unter Beispiel 1 erläutert, wurde diese Versuchsreihe mit der gleichen Ausgangsverbindung in gleicher Weise wiederholt. Es konnten die Werte des Beispiels 1 bestätigt werden. Die Wasserstoffaufnahme zeigte das gleiche Bild. Die Analyse zeigte, daß 47,5% des Ausgangsprodukts hydriert waren. 13,5% waren zum 1,4-Cyclohexan-cis-derivat, 8,4% zum trans-1,4-Cyclohexanderivat und 21,0% zu dem Cyclohex-1'-en-yl-1'-derivat hydriert worden.

Die Anreicherung des N[3-(4'-tert.-Butyl-cyclohex-1'-en-1'-yl)-2-methyl-propyl]-cis-2,6-dimethyl-morpholins (1) erfolgte durch fraktionierte Destillation. Zur Abtrennung der Begleitkomponenten ist eine Kolonne mit einer Trennstufenzahl von über 70 Trennstufen erforderlich. Die gesamten Begleitkomponenten können als niedriger siedend abgetrennt werden. Die gewünschte Verbindung reichert sich im nicht-destillierten Rückstand an. Da die Siedepunktsunterschiede sehr gering sind, kann eine Aussage über die Zusammensetzung nur mittels Gaschromatographie-Analyse erfolgen. Die Anreicherung kann in einer ersten Fraktionierung auf über 70% Reinheit erfolgen. Bei der zweiten Fraktionierung wird ein olefinisches Produkt (1) von 90% Reinheit erhalten. $Kp_{18\,Torr}$ 202° C.

Durch Neutralisation mit einer Chlorwasserstofflösung in Äthanol wird das Hydrochlorid mit einem Fp von 206°C erhalten. Die NMR- und $^{13}C$-Spektren zeigen das Vorliegen der Doppelbindung am C-Atom 1.

Die Verbindung hat folgende Formel:

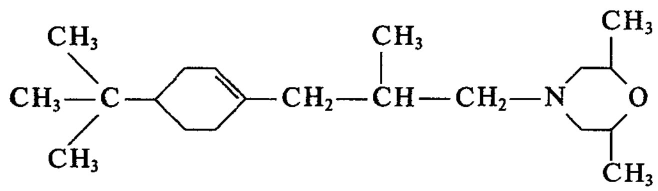

(1)

## Beispiel 3

### Synthese des N-[3-(4'-tert.Butylcyclohex-1'-en-1'yl)-2-methyl-1-propyl]-piperidin

a) In einen 3-l-Rollautoklaven werden 1200 g N-3-[(p.-tert.Butylphenyl)-2-methyl-1-propyl]-piperidin und 600 g Isopropanol eingefüllt. Als Hydrierkatalysator wird 1 g Ruthenium-oxyd-hydrat zugegeben. Der Autoklav wird druckdicht verschlossen und mit Stickstoff gespült. Anschließend wird auf 120° aufgeheizt und ein Wasserstoffdruck von 120 bar eingestellt. Im Verlauf von 7 Stunden kann eine Wasserstoffaufnahme von 200 bar erzielt werden. Die Hydrierung wird abgebrochen, obwohl die Hydrierreaktion nicht bis zur vollständigen Umsetzung mit Wasserstoff gebracht wurde. Der Reaktionsaustrag (1779 g) besaß nach der gaschromatographischen Analyse die folgende Zusammensetzung: (Das Lösungsmittel wurde nicht berücksichtigt.)

| | | | |
|---|---|---|---|
| Cyclohexan-derivat | cis | = | 18,48% |
| Cyclohexanderivat | trans | = | 16,21% |
| Cyclohexen-derivat | | = | 10,97% |
| nicht umgesetzte aromatische Ausgangsverbindung | | = | 54,34% |

### b) Anreicherung des Cyclohexenderivates

Über eine Destillationskolonne mit etwa 60 Trennstufen konnte bei der fraktionierten Destillation unter einem Druck von 3 Torr in den ersten Fraktionen ein bezüglich des cis-Cyclohexanderivats angereichertes Produkt erhalten werden.

In den weiteren Fraktionen wurde eine Anreicherung des nicht umgesetzten aromatischen Ausgangsproduktes erhalten. Am Schluß der Fraktionierung blieb als nichtdestilliertes Produkt ein bezüglich des Cyclohexenderivats angereichertes Produkt zurück. Es wurden 67 g dieses Produkts mit der folgenden Zusammensetzung erhalten:

| | | | |
|---|---|---|---|
| Cyclohexan-derivat | cis | = | 0% |
| Cyclohexan-derivat | trans | = | 10,32% |
| Cyclo-hexen-derivat | | = | 56,52% |
| Ausgangsprodukt (Aromat) | | = | 33,03% |

Das $^{13}$C-NMR und das NMR-Spektrum bestätigen diese Zusammensetzung. Sowohl im NMR-Spektrum als auch im C-NMR-Spektrum war das Vorhandensein nur einer olefinischen Verbindung festzustellen.

## Beispiel 4

### Synthese des N-[3(4'-tert.Butyl-cyclohex-1'-en-1-yl)-2-methyl-1-propyl]-pyrrolidin

In gleicher Weise wie unter Beispiel 3 beschrieben wurde, konnte bei der Umsetzung von 1500 g N(3-(p.tert.Butylphenyl)-2-methyl-propyl-1)-pyrrolidin mit Hilfe von Rutheniumoxydhydrat als Katalysator bei 120° unter einem Wasserstoffdruck von 120 bar im Verlauf von 23 Stunden eine Wasserstoffaufnahme von 200 bar erreicht werden.

Das Reaktionsprodukt besaß nach der gaschromatogaphischen Analyse die folgende Zusammensetzung:

| | | | |
|---|---|---|---|
| Cyclohexan-derivat | 1,4-cis | = | 25,39% |
| Cyclohexan-derivat | 1,4-trans | = | 18,36% |
| Cyclohexenderivat | | = | 12,46% |
| Ausgangsprodukt (Aromat) | | = | 39,90% |

Bei der fraktionierten Destillation über eine Kolonne mit circa 60 Trennstufen war es möglich in den anfänglich übergehenden Fraktionen das cis-Cyclohexanderivat anzureichern. In den dann folgenden Fraktionen trat eine Anreicherung des nicht umgesetzten aromatischen Ausgangsproduktes ein. Als Destillationsrückstand blieb ein stark angereichertes Cyclohexenderivat zurück. Der Destillationsrückstand wurde ohne Fraktionierung übergetrieben. Die gaschromatographische Analyse lieferte folgenden Befund:

| | | | |
|---|---|---|---|
| Cyclohexan-derivat | 1,4-cis | = | 0,0% |
| Cyclohexan-derivat | 1,4-trans | = | 14,65% |
| Cyclohexen-derivat | | = | 71,15% |
| Ausgangsprodukt (Aromat) | | = | 13,01% |
| höhersiedendes Nebenprodukt | | = | 1,19% |

Beispiel 5

In einem Rührautoklav mit einem Reaktionsvolumen von 17 Liter wurden 8000 g N[3-(p.tert.Butylphenyl)-2-methyl-1-propyl]-cis-2,6-dimethylmorpholin und 2000 g Isopropanol eingefüllt. Als Hydrierkatalysator wurden 3 g Rutheniumoxydhydrat zugegeben.

Die Hydrierung erfolgte bei 140° und einem Wasserstoffdruck von 140 bar. Es konnte unter diesen Bedingungen eine Wasserstoffaufnahme von 42 bar ermittelt werden. Um den Umsatz zu steigern, wurde bei 160° und 160 bar Wasserstoffdruck die Hydrierung fortgesetzt. Nach einer weiteren Wasserstoffaufnahme von 33 bar wurde die Reaktion abgebrochen. Das Reaktionsgemisch setzte sich nach der GC(Gaschromatographie)-Analyse wie folgt zusammen:

| | | |
|---|---|---|
| Cyclohexan-derivat | 1,4-cis | 19,01% |
| Cyclohexan-derivat | 1,4-trans | 13,77% |
| Cyclohexen-derivat | | 23,78% |
| Ausgangsprodukt (Aromat) | | 43,94% |

Die Anreicherung des Cyclohexen-Derivates erfolgte in gleicher Weise wie oben beschrieben durch fraktionierte Destillation. Wird bei 20 Torr gearbeitet, so kann ebenfalls ohne größere Verluste im Destillationsrückstand eine starke Anreicherung registriert werden. Werden 1600 g des in Beispiel 5 erhaltenen Produktes über eine Kolonne mit circa 60 Trennstufen fraktioniert und in Fraktionen von jeweils etwa 80 g geschnitten, so enthalten die ersten sechs Fraktionen (483 g) im Mittel nur 6,5% olefinisches Produkt. Die nächsten fünf Fraktionen (383 g) weisen einen mittleren Olefinanteil von 13% auf.

Der Destillationsrückstand (61 g) enthält neben 84% Cyclohexenderivat 6% Cyclohexan-trans-Derivat und 10% aromatisches Ausgangsprodukt.

Die Fraktionen gehen bei 207—210° bei einem Druck von 25 Torr über. Der Destillationsrückstand kann bei einem Druck von 0,3 Torr bei 148° überdestilliert werden.

Das $^{13}C$-NMR-Spektrum des übergetriebenen Destillationsrückstandes besitzt die nach der GC-Analyse zu erwartenden Signale für den Olefin-Aromaten und trans-Cyclohexan-Anteil. IR- und NMR-Spektren bestätigen die Zusammensetzung.

Durch Neutralisation dieser olefinreichen Fraktion mit Chlorwasserstoff in iso-Propylacetat kann ein Hydrochlorid mit einem Fp von 208—210° erhalten werden. Das Salz kann mit Natronlauge in die freie Base zurückverwandelt werden. Die $^{13}C$-NMR-Analyse zeigt, daß ein spektroskopisch reines Olefin vorliegt. Aromatenanteile und trans-Cyclohexananteile sind nicht mehr zu erkennen. Die H-NMR-spektroskopische Analyse bestätigt diesen Befund.

Die Fungizide sind insbesondere geeignet zur Bekämpfung von Pflanzenkrankheiten, z. B. Erysiphe graminis (echter Mehltau) an Getreide, Erysiphe cichoriacearum (echter Mehltau) an Kürbisgewächsen, Podosphaera leucotricha an Äpfeln, Uncinula necator an Reben, Erysiphe polygoni an Bohnen, Sphaerotheca pannosa an Rosen, Microsphaera querci an Eichen, Botrytis cinerea an Erdbeeren, Reben, Mycosphaerella musicola an Bananen, Puccinia-Arten (Rostpilze) an Getreide, Uromyces appendiculatus und U. phaseoli an Bohnen, Hemileia vastatrix an Kaffee und Rhizoctonia solani. Sie sind systematisch wirksam; sie werden sowohl über die Wurzeln als auch über die Blätter aufgenommen und im Pflanzengewebe transportiert.

Bei der Anwendung der Wirkstoffe zur Behandlung von Pflanzen gegen Pilzinfektionen liegen die Aufwandmengen zwischen 0,025 und 5 kg Wirkstoff/ha Fläche. Zum Oberflächenschutz von Bäumen oder Früchten können die Wirkstoffe auch in Verbindung mit Kunststoffdispersionen 0,25%ig bis 5%ig, bezogen auf das Gewicht der Dispersion, verwendet werden. Die Fungizide enthalten im allgemeinen zwischen 0,1 und 95 Gewichtsprozent Wirkstoff, vorzugsweise zwischen 0,5 und 90%.

Die Wirkstoffe können mit anderen bekannten Fungiziden gemischt werden. In vielen Fällen erhält man dabei eine Vergrößerung des fungiziden Wirkungsspektrums; bei einer Anzahl von Fungizidmischungen in den Gewichtsverhältnissen 1 : 10 bis 10 : 1 treten auch synergistische Effekte

auf, d. h., die fungizide Wirksamkeit des Kombinationsproduktes ist größer als die der addierten Wirksamkeiten der Einzelkomponenten. Fungizide, die mit den erfindungsgemäßen Verbindungen kombiniert werden können, sind beispielsweise:

Dithiocarbamate und deren Derivate, wie
Zinkdimethyldithiocarbamat,
Manganäthylenbisdithiocarbamat,
Mangan-Zink-äthylendiamin-bis-dithiocarbamat,
Zinkäthylenbisdithiocarbamat,
Tetramethylthiuramdisulfid,
Ammoniak-Komplex von Zink-(N,N-äthylen-bis-dithiocarbamat) und
N,N'-Polyäthylen-bis-(thiocarbamoyl)-disulfid,
Zink-(N,N'-propylen-bis-dithiocarbamat),
Ammoniak-Komplex von Zink-(N,N'-propylen-bis-dithiocarbamat) und
N,N'-Polypropylen-bis-(thiocarbamoyl)-disulfid;
heterocyclische Verbindungen, wie
N-Trichlormethylthio-tetrahydrophthalimid,
N-Trichlormethylthio-phthalimid,
N-(1,1,2,2-Tetrachloräthylthio)-tetrahydrophthalimid,
1-(Butylcarbamoyl)-2-benzimidazol-carbaminsäuremethylester,
2-Methyloxycarbonylamino-benzimidazol,
2,3-Dihydro-5-carboxanilido-6-methyl-1,4-oxathiin-4,4-dioxid,
2,3-Dihydro-5-carboxanilido-6-methyl-1,4-oxathiin,
5-Butyl-2-dimethylamino-4-hydroxy-6-methyl-pyrimidin,
1,2-Bis-(3-äthoxycarbonyl-2-thioureido)-benzol,
1,2-Bis-(3-methoxycarbonyl-2-thioureido)-benzol
und verschiedene andere Fungizide, wie
Dodecylguanidinacetat,
N-Dichlorfluormethylthio-N',N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Jodbenzoesäureanilid,
2-Brombenzoesäureanilid,
3-Nitro-isophthalsäure-diisopropylester,
1-(1,2,4-Triazolyl-1')-[1-(4'-chlorphenoxy)]-3,3-dimethylbutan-2-on,
1-(1-Imidazolyl)-2-allyloxy-2-(2,4-dichlorphenyl)-äthan,
Piperazin-1,4-diyl-bis-1-(2,2,2-Trichloräthyl)-formamid,
2,4,5,6-Tetrachlor-isophthalonitril,
1,2-Dimethyl-3,5-diphenyl-pyrazoliniummethylsulfat.

Die Anwendung erfolgt z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen, Beizen oder Gießen. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate zum Beispiel Methanol, Äthanol, Propanol, Butanol, Chloroform, Tetrachlorkohlenstoff, Cyclohexanol, Cyclohexanon, Chlorbenzol, Isophoron usw., stark polare Lösungsmittel, z. B. Dimethylformamid, Dimethylsulfoxid, N-Methylpyrrolidon, Wasser usw. in Betracht. Die Salze können in Form ihrer wäßrigen Lösungen verwendet werden.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen: Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sufatiertem Fettalkoholglykoläther, Kondensationsprodukte von

sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylen-octylphenoläther, äthoxyliertes Isooctylphenol-, Octylphenol-, Nonylphenol, Alkylphenolpolyglykoläther, Tributylphenylpolyglykoläther, Alkylarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Kieselsäuren, Silikate, Talkum, Kaolin, Kalk, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Zu den Mischungen oder Einzelwirkstoffen können Öle verschiedenen Typs, Herbizide, Fungizide, Nematozide, Insektizide, Bakterizide, Spurenelemente, Düngemittel, Antischaummittel (z. B. Silikone), Wachstumsregulatoren, Antidotmittel oder andere wirksame Verbindungen, zugesetzt werden.

Für den folgenden Versuch wurde die bekannte Verbindung N-(3-p-tert.-Butyl-phenyl-2-methyl-1-propyl)-cis-2,6-dimethylmorpholin als Vergleichssubstanz verwendet.

## Wirksamkeit gegen Weizenmehltau

Blätter von in Töpfen gewachsenen Weizenkeimlingen der Sorte »Jubilar« werden mit wäßrigen Emulsionen aus 80% (Gewichtsprozent) Wirkstoff und 20% Emulgiermittel besprüht und nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80% relativer Luftfeuchtigkeit aufgestellt. Nach 10 Tagen wird das Ausmaß der Mehltaupilzentwicklung ermittelt.

Das Ergebnis des Versuchs zeigt, daß der erfindungsgemäße Wirkstoff 1 eine bessere fungizide Wirkung als der bekannte Wirkstoff 2 hat.

## Beispiel I

Man vermischt 90 Gewichtsteile der Verbindung 1 mit 10 Gewichtsteilen N-Methyl-α-pyrrolidon und erhält eine Lösung, die zur Anwendung in Form kleinster Tropfen geeignet ist.

## Beispiel II

20 Gewichtsteile der Verbindung 1 werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-mono-äthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

## Beispiel III

20 Gewichtsteile der Verbindung 1 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

## Beispiel IV

20 Gewichtsteile der Verbindung 1 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

## Beispiel V

20 Gewichtsteile des Wirkstoffs 1 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

## Beispiel VI

3 Gewichtsteile der Verbindung 1 werden mit 97 Gewichtsteilen feinteiligem Kaolin innig vermischt. Man erhält auf diese Weise ein Stäubemittel, das 3 Gewichtsprozent des Wirkstoffs enthält.

## Beispiel VII

30 Gewichtsprozent der Verbindung 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

## Beispiel VIII

40 Gewichtsteile des Wirkstoffs 1 werden mit 10 Teilen Natriumsalz eines Phenolsulfonsäure-harnstoff-formaldehyd-kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

## Beispiel IX

20 Teile des Wirkstoffs 1 werden mit 12 Teilen Calciumsalz der Dodecylbenzolsulfonsäure, 8 Teile Fettalkohol-polyglykoläther, 2 Teilen Natriumsalz eines Phenolsulfonsäure-harnstoff-formaldehyd kondensats und 68 Teilen eines paraffinischen Mineralöls innig vermischt. Man erhält eine stabile ölige Dispersion.

**Patentansprüche für die Vertragsstaaten: BE, CH, DE, FR, GB, IT, LI, NL, SE**

1. N[3(4'-tert.Butylcyclohex-1'-en-1'-yl)-2-methyl-propyl]-cycloalkylamin der Formel

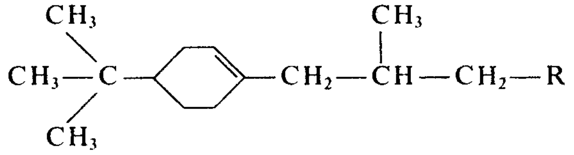

in der R den Rest des 2,6-Dimethylmorpholins, Piperidins oder Pyrrolidins bedeutet und seine Salze.

2. Verfahren zur Herstellung einer Verbindung gemäß Anspruch 1, dadurch gekennzeichnet, daß man N-[3-(para-tertiär-Butyl-phenyl)-2-methyl-1-propyl]-2,6-dimethyl-morpholin, -piperidin oder -pyrrolidin in Gegenwart eines Katalysators mit Wasserstoff partiell hydriert.

3. Fungizid, enthaltend eine Verbindung gemäß Anspruch 1.

4. Verfahren zur Bekämpfung von Pilzen, dadurch gekennzeichnet, daß man die vor Pilzbefall zu schützenden Gegenstände oder die Pilze behandelt mit einer Verbindung gemäß Anspruch 1.

**Patentansprüche für den Vertragsstaat: AT**

1. Fungizid enthaltend N[3(4′-tert.Butylcyclohex-1′-en-1′-yl)-1-methyl-propyl]-cycloalkylamin der Formel

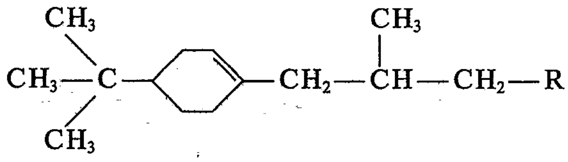

in der R den Rest des 2,6-Dimethylmorpholins, Piperidins oder Pyrrolidins bedeutet oder seine Salze.

2. Verfahren zur Herstellung einer Verbindung wie definiert in Anspruch 1, dadurch gekennzeichnet, daß man N-[3-(para-tertiär-Butyl-phenyl)-2-methyl-1-propyl]-2,6-dimethyl-morpholin, -piperidin oder -pyrrolidin, in Gegenwart eines Katalysators mit Wasserstoff partiell hydriert.

3. Verfahren zur Bekämpfung der Pilze, dadurch gekennzeichnet, daß man die vor Pilzbefall zu schützenden Gegenstände oder die Pilze behandelt mit einem Fungizid gemäß Anspruch 1.

**Claims for the Contracting states: BE, CH, DE, FR, GB, IT, LI, NL, SE**

1. N[3(4′-tert.-Butylcyclohex-1′-en-1′-yl)-2-methylpropyl]-cycloalkylamine of the formula

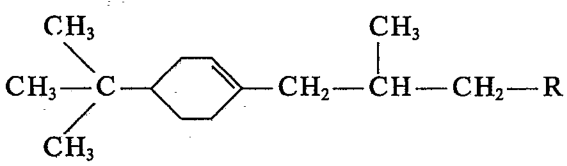

where R is the radical of 2,6-dimethylmorpholine, piperidine or pyrrolidone, and its salts.

2. A process for the preparation of a compound as claimed in claim 1, wherein N-[3-(para-tert.-butylphenyl)-2-methyl-1-propyl]-2,6-dimethylmorpholine, -piperidine or -pyrrolidine is partially hydrogenated with hydrogen in the presence of a catalyst.

3. A fungicide containing a compound as claimed in claim 1.

4. A process for combating fungi, wherein the objects to be protected against fungal attack or the fungi are treated with a compound as claimed in claim 1.

**Claims for the Contracting state: AT**

1. A fungicide containing N[3(4′-tert.-butylcyclohex-1′-en-1′-yl)-2-methylpropyl]-cycloalkylamine of the formula

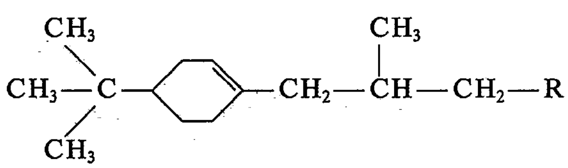

where R is the radical of 2,6-dimethylmorpholine, piperidine or pyrrolidone, or a salt thereof.

2. A process for the preparation of a compound as defined in claim 1, wherein N-[3-(para-tert.-butylphenyl)-2-methyl-1-propyl]-2,6-dimethylmorpholine, -piperidine or -pyrrolidine is partially hydrogenated with hydrogen in the presence of a catalyst.

3. A process for combating fungi, wherein the objects to be protected against fungal attack or the fungi are treated with a fungicide as claimed in claim 1.

**Revendications pour les Etats contractants: BE, CH, DE, FR, GB, IT, LI, NL, SE**

1. N-[3-(4′-tert.Butyl-cyclohex-1′-én-1′-yl)-2-méthyl-propyl]-cycloalkyl-amine de la formule

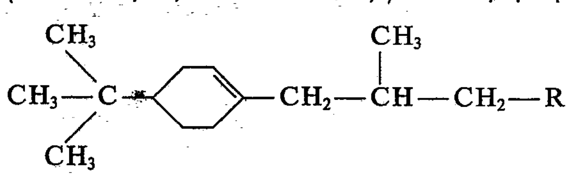

dans laquelle R représente un reste 2,6-diméthyl-morpholine ou pipéridine ou pyrrolidine, et ses sels.

2. Procédé de préparation d'un composé selon la revendication 1, caractérisé en ce que l'on soumet la N-[3-(para-tert.butyl-phényl)-2-méthyl-1-propyl]-2,6-diméthylmorpholine, -pipéridine ou -pyrrolidine à une hydrogénation partielle par l'hydrogène en présence d'un catalyseur.

3. Fongicide contenant un composé selon la revendication 1.

4. Procédé de lutte contre les champignons, caractérisé en ce que l'on traite les objets à protéger des champignons ou les champignons avec un composé selon la revendication 1.

**Revendications pour l'Etat contractant: AT**

1. Composition fongicide, contenant une N-[3-(4'-tert.butyl-cyclohex-1'-én-1'-yl)-2-méthyl-propyl]-cycloalkyl-amine de la formule

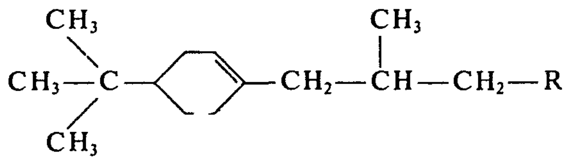

dans laquelle R représente un reste 2,6-diméthyl-morpholine ou pipéridine ou pyrrolidine, ou un sel de celle-ci.

2. Procédé de préparation d'un composé tel que défini dans la revendication 1, caractérisé en ce que l'on soumet la N-[3-(para-tert.butyl-phényl)-2-méthyl-1-propyl]-2,6-diméthyl-morpholine, -pipéridine ou -pyrrolidine à une hydrogénation partielle par l'hydrogène en présence d'un catalyseur.

3. Procédé de lutte contre les champignons, caractérisé en ce que l'on traite les objets à protéger des champignons ou les champignons avec un fongicide selon la revendication 1.